# EUROPEAN PATENT SPECIFICATION

(11) **EP 3 456 245 B1**
(45) Date of publication and mention of the grant of the patent: **08.12.2021**
(21) Application number: 17813624.8
(22) Date of filing: 16.06.2017
(51) Int. Cl.: A61B 5/00, A61B 5/053

(54) **PORTABLE DEVICE AND METHOD FOR MEASURING SKIN HYDRATION USING SAME**
TRAGBARE VORRICHTUNG UND VERFAHREN ZUR MESSUNG DER HAUTHYDRATION DAMIT
DISPOSITIF PORTABLE ET PROCÉDÉ DE MESURE D'HYDRATATION DE LA PEAU ASSOCIÉ

(30) Priority: 17.06.2016 KR 20160075927
(43) Date of publication of application: 20.03.2019
(73) Proprietor: Samsung Electronics Co., Ltd., Suwon-si, Gyeonggi-do 16677 (KR)
(72) Inventor: CHOI, Jongmin, Gyeonggi-do 16677 (KR); PARK, Minho, Gyeonggi-do 16677 (KR); LEE, Donghyun, Gyeonggi-do 16677 (KR); LEE, Jaesung, Gyeonggi-do 16677 (KR); LIM, Daehyeong, Gyeonggi-do 16677 (KR); CHOE, Keumsun, Gyeonggi-do 16677 (KR); CHOI, Wonsuk, Gyeonggi-do 16677 (KR)
(74) Representative: HGF
(86) International application number: PCT/KR2017/006299
(87) International publication number: WO 2017/217801

(56) References cited:
- JP-A- 2004 105 446
- KR-A- 20110 127 081
- KR-B1- 100 398 362
- KR-B1- 101 274 303
- US-A1- 2003 060 693
- US-A1- 2006 239 547
- US-A1- 2006 239 547
- US-A1- 2008 221 411
- US-A1- 2014 171 759

## Description

### [Technical Field]

Various embodiments of the present invention relate to a portable device and a method of measuring the degree of skin hydration using the same.

### [Background Art]

Skin is a structure made by combining two components called the epidermis and dermis. The skin surface has the property of flexibility and elasticity, and this is a major factor in protecting and allowing smooth movement of a human body. This property depends on the amount of moisture contained in the stratum corneum of the epidermis and is adjusted by a protective function and a water retention function of the stratum corneum.

In order to maintain a healthy skin, there is increased demand for determining a skin condition, and modern medicine is developing devices that can measure the degree of skin hydration by various methods.

A method that has been used to measure the degree of skin hydration is to measure the moisture of the stratum corneum of the epidermis using electric conductivity. Because the stratum corneum contains a large amount of electrolytes such as salts and amino acids, the method uses the principle that the skin surface has an electrically conductive property in the presence of moisture.

### [Disclosure of Invention]

### [Technical Problem]

However, this method may measure only moisture information of an epidermal layer of an outer layer of the skin, and it may not be much help in making a substantial determination of the state of the skin.

US 2014/171759 discloses an apparatus and method for non-invasive determination of hydration, hydration state, total body water, or water concentration by quantitative spectroscopy. The system includes subsystems optimized to contend with the complexities of the tissue spectroscopy, high signal-to-noise ratio and photometric accuracy requirements, tissue sampling errors, calibration maintenance, and calibration transfer. The subsystems include an illumination subsystem, a tissue sampling subsystem, a spectrometer subsystem, a data acquisition subsystem, a computing subsystem, and a calibration subsystem.

US 2006/239547 relates to the use of optical skin measurements to determine skin properties such as surface topography, hydration, elasticity, pigmentation intensity or uniformity, dermal thickness, dermal perfusion, presence or concentration or composition of oil on or near the surface, and apparent age of the skin. It discloses a skin positioning system for positioning the skin relative to other parts of the apparatus. An illumination system, adapted to produce illumination radiation, can mount with the skin positioning system such that illumination radiation impinges on a first portion of the skin at a first determined angle thereto. A detection system can mount with the skin positioning system such that radiation from a second portion of the skin at a second determined angle therefrom impinges on the detector. An analysis system can receive information from the detection system, and determine one or more skin properties from the detected radiation and the illumination radiation.

US 2008/221411 discloses a system and method for determining tissue hydration. The method includes transmitting electromagnetic radiation at tissue and detecting the absorption spectrum of the tissue using a spectrum analyzer located in a sensor. Further, the method includes providing a signal correlative to the absorption spectrum from the spectrum analyzer to a monitor and processing the signal to determine an amount of water content in the tissue.

US 2003/060693 discloses an apparatus and method for non-destructively estimating a tissue property, such as hydration, of a living subject utilizes in vivo spectral measurements made by irradiating skin tissue with near infrared (NIR) light. The apparatus includes a spectroscopic instrument in conjunction with a subject interface. The resulting spectra are passed to an analyzer for further processing, which includes detecting and eliminating invalid spectral measurements, and preprocessing to increase the signal-to-noise ratio. Finally, an estimation model developed from an exemplary set of measurements is applied to predict the tissue hydration for the sample. The method of tissue hydration measurement provides additional information about primary sources of systematic tissue variability, namely, the water content of the epidermal layer of skin and the penetration depth of the incident light. Tissue hydration measurement is therefore suitable for further spectral analysis and quantification of biological and chemical compounds, such as analytes.

Various embodiments of the present invention may provide a method and portable device that can correct an influence of contamination of a skin layer, external temperature and humidity, and the like.

Various embodiments of the present invention may provide a method and portable device that can easily measure the degree of hydration using a portable device.

### [Solution to Problem]

In accordance with an aspect of the present invention, there is provided a portable device according to claim 1.

In accordance with another aspect of the present invention, there is provided a method of measuring the degree of hydration of a skin of a user using a portable device according to claim 1, the method according to claim 9. Further, in accordance with another aspect of the present invention, there is provided a computer program according to claim 10.

### [Advantageous Effects of Invention]

A portable device and method according to various embodiments of the present invention can measure a moisture value of a specific object (e.g., user's skin) using a portable device including a spectroscopic sensor. Further, the portable device and method can further measure a moisture value of a skin layer of a user's skin using at least one method and can obtain an accurate moisture value of a dermal layer of the user's skin using the same. Thereby, a moisture value of the user's skin can be determined and related services can be provided.

### [BRIEF Description of Drawings]

FIG. 1 is a block diagram illustrating a configuration of an electronic device in a network environment according to various embodiments;
FIG. 2 is a block diagram illustrating a configuration of an electronic device according to various embodiments;
FIG. 3 is a block diagram illustrating a configuration of a program module according to various embodiments;
FIG. 4 is a block diagram illustrating a configuration of a portable device according to various embodiments;
FIG. 5 is a cross-sectional view illustrating a spectroscopic sensor according to various embodiments;
FIG. 6A is a cross-sectional view illustrating a Fabry-Perot Interferometer (FPI) filter according to an embodiment of a light splitter;
FIG. 6B is a cross-sectional view illustrating a linear variable (LV) filter according to an embodiment of a light splitter;
FIGS. 7A to 7C are diagrams illustrating a portable device including a spectroscopic sensor according to an embodiment;
FIG. 8 is a flowchart illustrating a method of measuring the degree of hydration of an object according to various embodiments;
FIG. 9 is a graph illustrating a prediction model of an absorption spectrum according to the degree of hydration of an object according to various embodiments;
FIG. 10 is a flowchart illustrating a method of determining a second degree of hydration of an object using electrical conductivity of an object surface according to various embodiments not covered by the present invention;
FIG. 11 is a flowchart illustrating a method of determining a second degree of hydration of an object using characteristics of different wavelength bands according to various embodiments;
FIG. 12 is a flowchart illustrating a method of determining a second degree of hydration of an object using a polarizing film according to various embodiments not covered by the present invention;
FIG. 13 is a flowchart illustrating a method of determining a third degree of hydration in a portable device excluding a light splitter according to various embodiments not covered by the present invention;
FIG. 14 is a flowchart illustrating a method of providing a personalized user interface (UI) using a determined third degree of hydration according to various embodiments; and
FIGS. 15A and 15B are diagrams illustrating a personalized UI according to various embodiments.

### [Mode for the Invention]

Hereinafter, various embodiments of the present disclosure may be described with reference to accompanying drawings. Accordingly, those of ordinary skill in the art will recognize that modification, and/or alternative on the various embodiments described herein can be variously made without departing from the scope of the present disclosure as defined by the appended claims. With regard to description of drawings, similar elements may be marked by similar reference numerals. The terms of a singular form may include plural forms unless otherwise specified. In this disclosure, the expressions "A or B", "at least one of A or/and B", or "one or more of A or/and B", and the like may include any and all combinations of one or more of the associated listed items. The terms, such as "first", "second", and the like may be used to refer to various elements regardless of the order and/or the priority and to distinguish the relevant elements from other elements, but do not limit the elements. When an element (e.g., a first element) is referred to as being "(operatively or communicatively) coupled with/to" or "connected to" another element (e.g., a second element), the element may be directly coupled with/to or connected to the other element or an intervening element (e.g., a third element) may be present.

According to the situation, the expression "configured to" used in this disclosure may be used as, for example, the expression "suitable for", "having the capacity to", "adapted to", "made to", "capable of', or "designed to" in hardware or software. The expression "a device configured to" may mean that the device is "capable of' operating together with another device or other components. For example, a "processor configured to (or set to) perform A, B, and C" may mean a dedicated processor (e.g., an embedded processor) for performing a corresponding operation or a generic-purpose processor (e.g., a central processing unit (CPU) or an application processor) which performs corresponding operations by executing one or more software programs which are stored in a memory device.

An electronic device according to various embodiments of this disclosure may include at least one of, for example, smartphones, tablet personal computers (PCs), mobile phones, video telephones, electronic book readers, desktop PCs, laptop PCs, netbook computers, workstations, servers, personal digital assistants (PDAs), portable multimedia players (PMPs), Motion Picture Experts Group (MPEG-1 or MPEG-2) Audio Layer 3 (MP3) players, medical devices, cameras, or wearable devices. According to various embodiments, the wearable device may include at least one of an accessory type (e.g., watches, rings, bracelets, anklets, necklaces, glasses, contact lens, or head-mounted-devices (HMDs), a fabric or garment-integrated type (e.g., an electronic apparel), a body-attached type (e.g., a skin pad or tattoos), or a bio-implantable type (e.g., an implantable circuit). According to various embodiments, the electronic device may include at least one of, for example, televisions (TVs), digital versatile disc (DVD) players, audios, refrigerators, air conditioners, cleaners, ovens, microwave ovens, washing machines, air cleaners, set-top boxes, home automation control panels, security control panels, media boxes (e.g., Samsung HomeSync^{™}, Apple TV^{™}, or Google TV^{™}), game consoles (e.g., Xbox^{™} or PlayStation^{™}), electronic dictionaries, electronic keys, camcorders, electronic picture frames, and the like.

According to another embodiment, an electronic device may include at least one of various medical devices (e.g., various portable medical measurement devices (e.g., a blood glucose monitoring device, a heartbeat measuring device, a blood pressure measuring device, a body temperature measuring device, and the like), a magnetic resonance angiography (MRA), a magnetic resonance imaging (MRI), a computed tomography (CT), scanners, and ultrasonic devices), navigation devices, Global Navigation Satellite System (GNSS), event data recorders (EDRs), flight data recorders (FDRs), vehicle infotainment devices, electronic equipment for vessels (e.g., navigation systems and gyrocompasses), avionics, security devices, head units for vehicles, industrial or home robots, drones, automatic teller's machines (ATMs), points of sales (POSs) of stores, or internet of things (e.g., light bulbs, various sensors, sprinkler devices, fire alarms, thermostats, street lamps, toasters, exercise equipment, hot water tanks, heaters, boilers, and the like). According to an embodiment, the electronic device may include at least one of parts of furniture or buildings/structures, electronic boards, electronic signature receiving devices, projectors, or various measuring instruments (e.g., water meters, electricity meters, gas meters, or wave meters, and the like). According to various embodiments, the electronic device may be a flexible electronic device or a combination of two or more above-described devices. Furthermore, an electronic device according to an embodiment of this disclosure may not be limited to the above-described electronic devices. In this disclosure, the term "user" may refer to a person who uses an electronic device or may refer to a device (e.g., an artificial intelligence electronic device) that uses the electronic device.

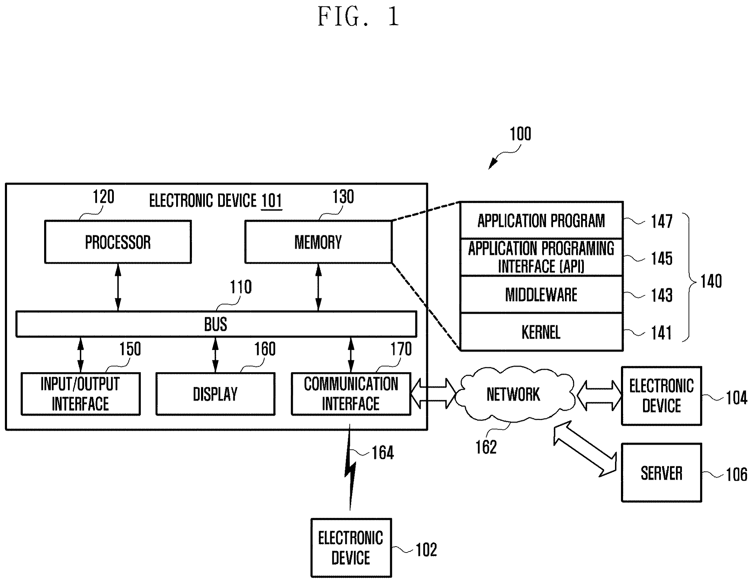

Referring to FIG. 1, according to various embodiments, an electronic device 101 in a network environment is described. The electronic device 101 may include a bus 110, a processor 120, a memory 130, an input/output interface 150, a display 160, and a communication interface 170. According to an embodiment, the electronic device 101 may not include at least one of the above-described elements or may further include other element(s).

The bus 110 may interconnect the above-described elements 110 to 170 and may include a circuit for conveying communications (e.g., a control message and/or data) among the above-described elements. The processor 120 may include one or more of a central processing unit (CPU), an application processor (AP), or a communication processor (CP).

For example, the processor 120 may perform an arithmetic operation or data processing associated with control and/or communication of at least other elements of the electronic device 101.

The memory 130 may include a volatile and/or nonvolatile memory. For example, the memory 130 may store instructions or data associated with at least one other element(s) of the electronic device 101. According to an embodiment, the memory 130 may store software and/or a program 140. The program 140 may include, for example, a kernel 141, a middleware 143, an application programming interface (API) 145, and/or an application program (or "an application") 147. At least a part of the kernel 141, the middleware 143, or the API 145 may be referred to as an "operating system (OS)". For example, the kernel 141 may control or manage system resources (e.g., the bus 110, the processor 120, the memory 130, and the like) that are used to execute operations or functions of other programs (e.g., the middleware 143, the API 145, and the application program 147). Furthermore, the kernel 141 may provide an interface that allows the middleware 143, the API 145, or the application program 147 to access discrete elements of the electronic device 101 so as to control or manage system resources.

The middleware 143 may perform, for example, a mediation role such that the API 145 or the application program 147 communicates with the kernel 141 to exchange data. Furthermore, the middleware 143 may process one or more task requests received from the application program 147 according to a priority. For example, the middleware 143 may assign the priority, which makes it possible to use a system resource (e.g., the bus 110, the processor 120, the memory 130, or the like) of the electronic device 101, to at least one of the application program 147 and may process the one or more task requests. The API 145 may be an interface through which the application program 147 controls a function provided by the kernel 141 or the middleware 143, and may include, for example, at least one interface or function (e.g., a command or an instruction) for a file control, a window control, image processing, a character control, or the like.

The input/output interface 150 may transmit an instruction or data input from a user or another external device, to other element(s) of the electronic device 101 or may output an instruction or data, received from other element(s) of the electronic device 101, to a user or another external device.

The display 160 may include, for example, a liquid crystal display (LCD), a light-emitting diode (LED) display, an organic LED (OLED) display, a microelectromechanical systems (MEMS) display, or an electronic paper display. The display 160 may display, for example, various contents (e.g., a text, an image, a video, an icon, a symbol, and the like) to a user. The display 160 may include a touch screen and may receive, for example, a touch, gesture, proximity, or hovering input using an electronic pen or a part of a user's body.

For example, the communication interface 170 may establish communication between the electronic device 101 and an external device (e.g., the first electronic device 102, the second electronic device 104, or the server 106). For example, the communication interface 170 may be connected to the network 162 over wireless communication or wired communication to communicate with the external device (e.g., the second electronic device 104 or the server 106).

For example, the wireless communication may include cellular communication using at least one of long-term evolution (LTE), LTE Advanced (LTE-A), Code Division Multiple Access (CDMA), Wideband CDMA (WCDMA), Universal Mobile Telecommunications System (UMTS), Wireless Broadband (WiBro), Global System for Mobile Communications (GSM), or the like. The wireless communication may include at least one of wireless fidelity (Wi-Fi), Bluetooth, Bluetooth low energy (BLE), Zigbee, near field communication (NFC), magnetic stripe transmission (MST), radio frequency (RF), a body area network, or the like. According to an embodiment, the wireless communication may include GNSS. The GNSS may be one of, for example, a global positioning system (GPS), a global navigation satellite system (Glonass), a Beidou navigation satellite system (hereinafter referred to as "Beidou"), or an European global satellite-based navigation system (hereinafter referred to as "Galileo"). Hereinafter, in this disclosure, "GPS" and "GNSS" may be interchangeably used. The wired communication may include at least one of, for example, a universal serial bus (USB), a high definition multimedia interface (HDMI), a recommended standard-232 (RS-232), power line communication, a plain old telephone service (POTS), or the like. The network 162 may include at least one of telecommunications networks, for example, a computer network (e.g., LAN or WAN), an Internet, or a telephone network.

Each of the first and second external electronic devices 102 and 104 may be a device of which the type is different from or the same as that of the electronic device 101. According to various embodiments, all or a portion of operations that the electronic device 101 will perform may be executed by another or plural electronic devices (e.g., the first electronic device 102, the second electronic device 104 or the server 106). According to an embodiment, in the case where the electronic device 101 executes any function or service automatically or in response to a request, the electronic device 101 may not perform the function or the service internally, but, alternatively additionally, it may request at least a portion of a function associated with the electronic device 101 at other electronic device (e.g., the electronic device 102 or 104 or the server 106). The other electronic device (e.g., the electronic device 102 or 104 or the server 106) may execute the requested function or additional function and may transmit the execution result to the electronic device 101. The electronic device 101 may provide the requested function or service using the received result or may additionally process the received result to provide the requested function or service. To this end, for example, cloud computing, distributed computing, or client-server computing may be used.

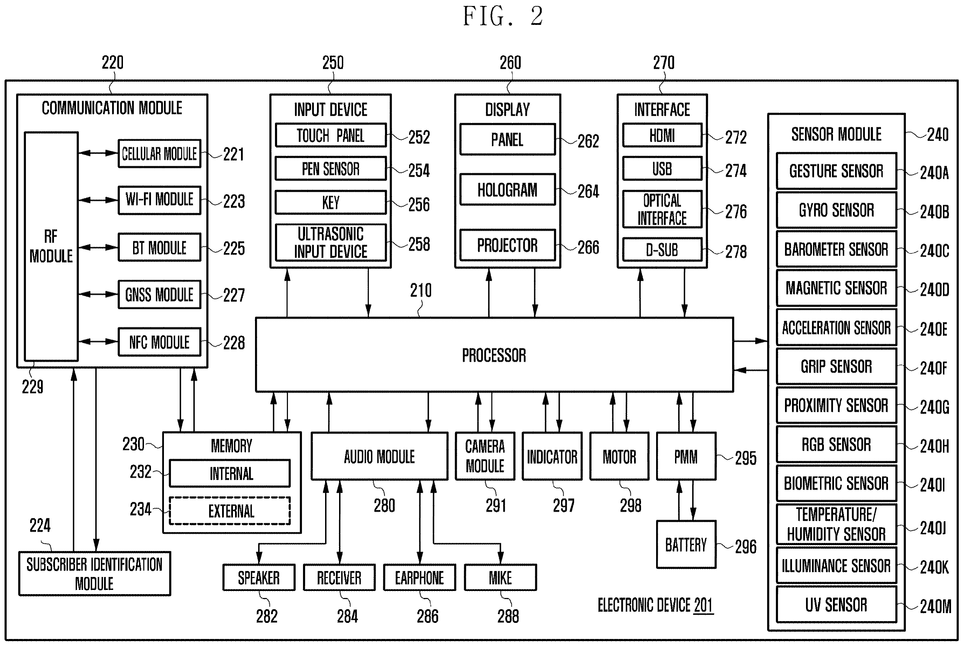

FIG. 2 illustrates a block diagram of an electronic device, according to various embodiments. An electronic device 201 may include, for example, all or a part of the electronic device 101 illustrated in FIG. 1. The electronic device 201 may include one or more processors (e.g., an application processor (AP)) 210, a communication module 220, a subscriber identification module 224, a memory 230, a sensor module 240, an input device 250, a display 260, an interface 270, an audio module 280, a camera module 291, a power management module 295, a battery 296, an indicator 297, and a motor 298. For example, the processor 210 may be implemented with a System on Chip (SoC). According to an embodiment, the processor 210 may further include a graphic processing unit (GPU) and/or an image signal processor. The processor 210 may include at least a part (e.g., a cellular module 221) of elements illustrated in FIG. 2.

The processor 210 may load an instruction or data, which is received from at least one of other elements (e.g., a nonvolatile memory), into a volatile memory and process the loaded instruction or data. The processor 210 may store result data in the nonvolatile memory.

The communication module 220 may be configured the same as or similar to the communication interface 170 of FIG. 1. The communication module 220 may include the cellular module 221, a Wi-Fi module 223, a Bluetooth (BT) module 225, a GNSS module 227, a near field communication (NFC) module 228, and a radio frequency (RF) module 229. The cellular module 221 may provide, for example, voice communication, video communication, a character service, an Internet service, or the like over a communication network. According to an embodiment, the cellular module 221 may perform discrimination and authentication of the electronic device 201 within a communication network by using the subscriber identification module (e.g., a SIM card) 224. According to an embodiment, the cellular module 221 may perform at least a portion of functions that the processor 210 provides. According to an embodiment, the cellular module 221 may include a communication processor (CP). According to an embodiment, at least a part (e.g., two or more) of the cellular module 221, the Wi-Fi module 223, the BT module 225, the GNSS module 227, or the NFC module 228 may be included within one Integrated Circuit (IC) or an IC package. For example, the RF module 229 may transmit and receive a communication signal (e.g., an RF signal). For example, the RF module 229 may include a transceiver, a power amplifier module (PAM), a frequency filter, a low noise amplifier (LNA), an antenna, or the like. According to another embodiment, at least one of the cellular module 221, the Wi-Fi module 223, the BT module 225, the GNSS module 227, or the NFC module 228 may transmit and receive an RF signal through a separate RF module. The subscriber identification module 224 may include, for example, a card and/or embedded SIM that includes a subscriber identification module and may include unique identify information (e.g., integrated circuit card identifier (ICCID)) or subscriber information (e.g., international mobile subscriber identity (IMSI)).

The memory 230 (e.g., the memory 130) may include an internal memory 232 or an external memory 234. For example, the internal memory 232 may include at least one of a volatile memory (e.g., a dynamic random access memory (DRAM), a static RAM (SRAM), a synchronous DRAM (SDRAM), or the like), a nonvolatile memory (e.g., a one-time programmable read only memory (OTPROM), a programmable ROM (PROM), an erasable and programmable ROM (EPROM), an electrically erasable and programmable ROM (EEPROM), a mask ROM, a flash ROM, a flash memory, a hard drive, or a solid state drive (SSD). The external memory 234 may include a flash drive such as compact flash (CF), secure digital (SD), micro secure digital (Micro-SD), mini secure digital (Mini-SD), extreme digital (xD), a multimedia card (MMC), a memory stick, or the like. The external memory 234 may be operatively and/or physically connected to the electronic device 201 through various interfaces.

The sensor module 240 may measure, for example, a physical quantity or may detect an operation state of the electronic device 201. The sensor module 240 may convert the measured or detected information to an electric signal. For example, the sensor module 240 may include at least one of a gesture sensor 240A, a gyro sensor 240B, a barometric pressure sensor 240C, a magnetic sensor 240D, an acceleration sensor 240E, a grip sensor 240F, the proximity sensor 240G, a color sensor 240H (e.g., red, green, blue (RGB) sensor), a biometric sensor 2401, a temperature/humidity sensor 240J, an illuminance sensor 240K, or an UV sensor 240M. Although not illustrated, additionally or generally, the sensor module 240 may further include, for example, an E-nose sensor, an electromyography (EMG) sensor, an electroencephalogram (EEG) sensor, an electrocardiogram (ECG) sensor, an infrared (IR) sensor, an iris sensor, and/or a fingerprint sensor. The sensor module 240 may further include a control circuit for controlling at least one or more sensors included therein. According to an embodiment, the electronic device 201 may further include a processor that is a part of the processor 210 or independent of the processor 210 and is configured to control the sensor module 240. The processor may control the sensor module 240 while the processor 210 remains at a sleep state.

The input device 250 may include, for example, a touch panel 252, a (digital) pen sensor 254, a key 256, or an ultrasonic input unit 258. For example, the touch panel 252 may use at least one of capacitive, resistive, infrared and ultrasonic detecting methods. Also, the touch panel 252 may further include a control circuit. The touch panel 252 may further include a tactile layer to provide a tactile reaction to a user. The (digital) pen sensor 254 may be, for example, a part of a touch panel or may include an additional sheet for recognition. The key 256 may include, for example, a physical button, an optical key, or a keypad. The ultrasonic input device 258 may detect (or sense) an ultrasonic signal, which is generated from an input device, through a microphone (e.g., a microphone 288) and may check data corresponding to the detected ultrasonic signal.

The display 260 (e.g., the display 160) may include a panel 262, a hologram device 264, a projector 266, and/or a control circuit for controlling the panel 262, the hologram device 264, or the projector 266. The panel 262 may be implemented, for example, to be flexible, transparent or wearable. The panel 262 and the touch panel 252 may be integrated into a single module. According to an embodiment, the panel 262 may include a pressure sensor (or force sensor) that measures the intensity of touch pressure by a user. The pressure sensor may be implemented integrally with the touch panel 252, or may be implemented as at least one sensor separately from the touch panel 252. The hologram device 264 may display a stereoscopic image in a space using a light interference phenomenon. The projector 266 may project light onto a screen so as to display an image. For example, the screen may be arranged in the inside or the outside of the electronic device 201. The interface 270 may include, for example, a high-definition multimedia interface (HDMI) 272, a universal serial bus (USB) 274, an optical interface 276, or a D-subminiature (D-sub) 278. The interface 270 may be included, for example, in the communication interface 170 illustrated in FIG. 1. Additionally or generally, the interface 270 may include, for example, a mobile high definition link (MHL) interface, a SD card/multi-media card (MMC) interface, or an infrared data association (IrDA) standard interface.

The audio module 280 may convert a sound and an electric signal in dual directions. At least a part of the audio module 280 may be included, for example, in the input/output interface 150 illustrated in FIG. 1. The audio module 280 may process, for example, sound information that is input or output through a speaker 282, a receiver 284, an earphone 286, or the microphone 288. For example, the camera module 291 may shoot a still image or a video. According to an embodiment, the camera module 291 may include at least one or more image sensors (e.g., a front sensor or a rear sensor), a lens, an image signal processor (ISP), or a flash (e.g., an LED or a xenon lamp). The power management module 295 may manage, for example, power of the electronic device 201. According to an embodiment, a power management integrated circuit (PMIC), a charger IC, or a battery or fuel gauge may be included in the power management module 295. The PMIC may have a wired charging method and/or a wireless charging method. The wireless charging method may include, for example, a magnetic resonance method, a magnetic induction method or an electromagnetic method and may further include an additional circuit, for example, a coil loop, a resonant circuit, a rectifier, or the like. The battery gauge may measure, for example, a remaining capacity of the battery 296 and a voltage, current or temperature thereof while the battery is charged. The battery 296 may include, for example, a rechargeable battery and/or a solar battery.

The indicator 297 may display a specific state of the electronic device 201 or a part thereof (e.g., the processor 210), such as a booting state, a message state, a charging state, and the like. The motor 298 may convert an electrical signal into a mechanical vibration and may generate the following effects: vibration, haptic, and the like. The electronic device 201 may include a processing device (e.g., a GPU) for supporting a mobile TV. The processing device for supporting the mobile TV may process media data according to the standards of digital multimedia broadcasting (DMB), digital video broadcasting (DVB), MediaFLO^{™}, or the like. Each of the above-mentioned elements of the electronic device according to various embodiments of the present disclosure may be configured with one or more components, and the names of the elements may be changed according to the type of the electronic device. In various embodiments, some elements of the electronic device (e.g., the electronic device 201) may be omitted or other additional elements may be added. Furthermore, some of the elements of the electronic device may be combined with each other so as to form one entity, so that the functions of the elements may be performed in the same manner as before the combination.

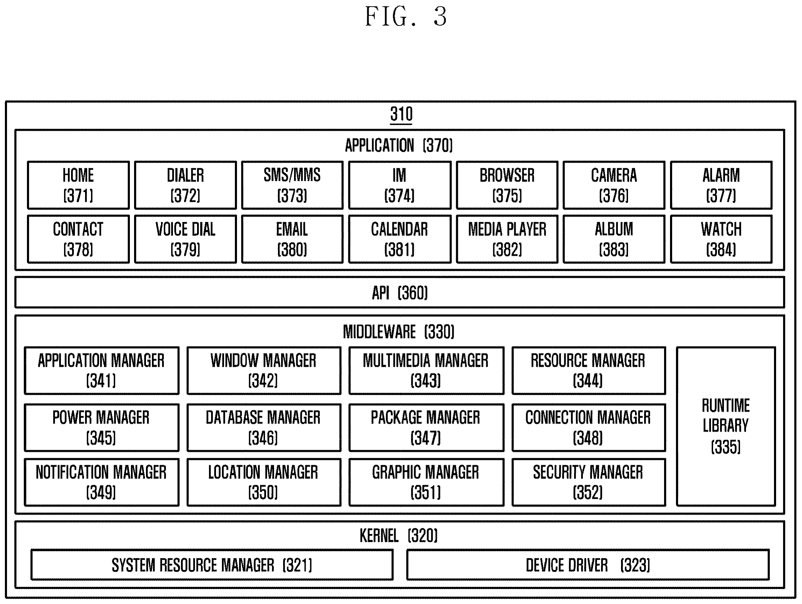

FIG. 3 illustrates a block diagram of a program module, according to various embodiments. According to an embodiment, a program module 310 (e.g., the program 140) may include an operating system (OS) to control resources associated with an electronic device (e.g., the electronic device 101), and/or diverse applications (e.g., the application program 147) driven on the OS. The OS may be, for example, Android^{™}, iOS^{™}, Windows^{™}, Symbian^{™}, Tizen^{™}, or Bada^{™}. The program module 310 may include a kernel 320 (e.g., the kernel 141), a middleware 330 (e.g., the middleware 143), an application programming interface (API) 360 (e.g., the API 145), and/or an application 370 (e.g., the application program 147). At least a portion of the program module 310 may be preloaded on an electronic device or may be downloadable from an external electronic device (e.g., the first electronic device 102, the second electronic device 104, the server 106, or the like).

The kernel 320 (e.g., the kernel 141) may include, for example, a system resource manager 321 or a device driver 323. The system resource manager 321 may control, allocate, or retrieve system resources. According to an embodiment, the system resource manager 321 may include a process managing unit, a memory managing unit, a file system managing unit, or the like. The device driver 323 may include, for example, a display driver, a camera driver, a Bluetooth driver, a shared memory driver, a USB driver, a keypad driver, a Wi-Fi driver, an audio driver, or an inter-process communication (IPC) driver. The middleware 330 may provide, for example, a function that the application 370 needs in common, or may provide diverse functions to the application 370 through the API 360 to allow the application 370 to efficiently use limited system resources of the electronic device. According to an embodiment, the middleware 330 may include at least one of a runtime library 335, an application manager 341, a window manager 342, a multimedia manager 343, a resource manager 344, a power manager 345, a database manager 346, a package manager 347, a connectivity manager 348, a notification manager 349, a location manager 350, a graphic manager 351, or a security manager 352.

The runtime library 335 may include, for example, a library module that is used by a compiler to add a new function through a programming language while the application 370 is being executed. The runtime library 335 may perform input/output management, memory management, or capacities about arithmetic functions. The application manager 341 may manage, for example, a life cycle of at least one application of the application 370. The window manager 342 may manage a graphic user interface (GUI) resource that is used in a screen. The multimedia manager 343 may identify a format necessary for playing diverse media files, and may perform encoding or decoding of media files by using a codec suitable for the format. The resource manager 344 may manage resources such as a memory space or source code of the application 370. The power manager 345 may manage a battery or power, and may provide power information for an operation of an electronic device. According to an embodiment, the power manager 345 may operate with a basic input/output system (BIOS). The database manager 346 may generate, search for, or modify database that is to be used in the application 370. The package manager 347 may install or update an application that is distributed in the form of package file.

The connectivity manager 348 may manage, for example, wireless connection. The notification manager 349 may provide an event, for example, arrival message, appointment, or proximity notification to a user. For example, the location manager 350 may manage location information about an electronic device. The graphic manager 351 may manage a graphic effect that is provided to a user, or manage a user interface relevant thereto. The security manager 352 may provide, for example, system security or user authentication. According to an embodiment, the middleware 330 may include a telephony manager for managing a voice or video call function of the electronic device or a middleware module that combines diverse functions of the above-described elements. According to an embodiment, the middleware 330 may provide a module specialized to each OS kind to provide differentiated functions. Additionally, the middleware 330 may dynamically remove a part of the preexisting elements or may add new elements thereto. The API 360 may be, for example, a set of programming functions and may be provided with a configuration that is variable depending on an OS. For example, in the case where an OS is the android or the iOS, it may provide one API set per platform. In the case where an OS is the tizen, it may provide two or more API sets per platform.

The application 370 may include, for example, applications such as a home 371, a dialer 372, an SMS/MMS 373, an instant message (IM) 374, a browser 375, a camera 376, an alarm 377, a contact 378, a voice dial 379, an e-mail 380, a calendar 381, a media player 382, an album 383, a watch 384, health care (e.g., measuring an exercise quantity, blood sugar, or the like) or offering of environment information (e.g., information of barometric pressure, humidity, temperature, or the like). According to an embodiment, the application 370 may include an information exchanging application to support information exchange between an electronic device and an external electronic device. The information exchanging application may include, for example, a notification relay application for transmitting specific information to an external electronic device, or a device management application for managing the external electronic device. For example, the notification relay application may include a function of transmitting notification information, which arise from other applications, to an external electronic device or may receive, for example, notification information from an external electronic device and provide the notification information to a user. The device management application may install, delete, or update for example, a function (e.g., turn-on/turn-off of an external electronic device itself (or a part of components) or adjustment of brightness (or resolution) of a display) of the external electronic device which communicates with the electronic device, and an application running in the external electronic device. According to an embodiment, the application 370 may include an application (e.g., a health care application of a mobile medical device) that is assigned in accordance with an attribute of an external electronic device. According to an embodiment, the application 370 may include an application that is received from an external electronic device. At least a portion of the program module 310 may be implemented by software, firmware, hardware (e.g., the processor 210), or a combination (e.g., execution) of two or more thereof, and may include modules, programs, routines, sets of instructions, processes, or the like for performing one or more functions.

The "module" used in this document may include a unit including hardware, software or firmware and may be interchangeably used with a term, for example, logic, a logical block, a part or a circuit. The "module" may be an integrated part, a minimum unit to perform one or more functions, or a part thereof. The "module" may be implemented mechanically or electronically, and may include an application-specific integrated circuit (ASIC) chip, field-programmable gate arrays (FPGAs) or a programmable logic device which performs some operations and which has been known or is to be developed, for example. At least some of a device (e.g., modules or functions thereof) or method (e.g., operations) according to various embodiments may be implemented as instructions stored in a computer-readable storage medium (e.g., the memory 130) in the form of a program module. If the instructions are executed by a processor (e.g., the processor 120), the processor may perform functions corresponding to the instructions. The computer-readable storage medium may include a hard disk, a floppy disk, magnetic media (e.g., magnetic tape), optical media (e.g., CD-ROM), a DVD, magneto-optical media (e.g., a floptical disk), and embedded memory. The instructions may include code generated by a compiler or code executable by an interpreter. The module or program module according to various embodiments may include at least one of the aforementioned elements, may omit some of the elements or may further include other elements. Operations performed by the module, program module or other elements according to various embodiments may be executed in a sequential, parallel, repetitive or heuristic manner or at least some operations may be executed in a different sequence or omitted or may further include other operations.

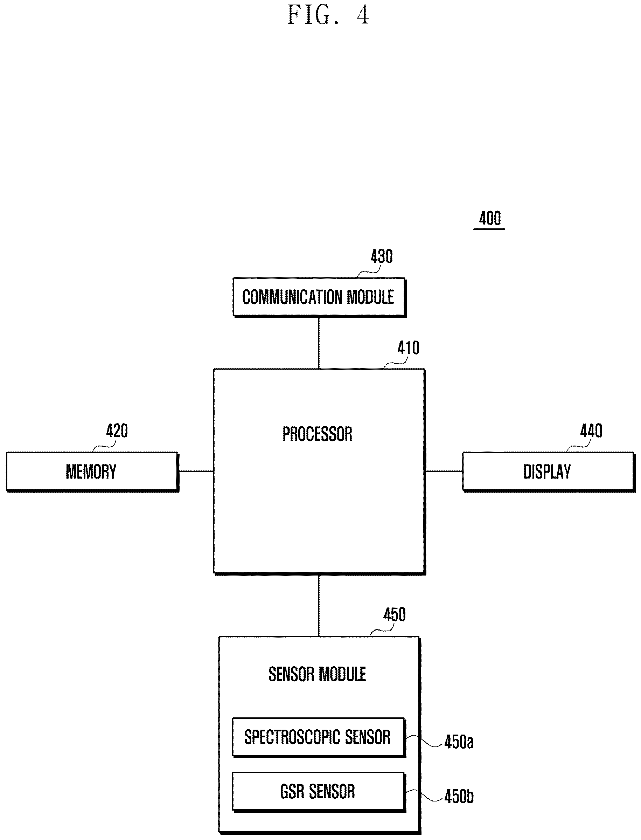

FIG. 4 is a block diagram illustrating a configuration of a portable device 400 according to various embodiments.

According to various embodiments, the portable device 400 may include at least one processor 410 (e.g., an AP), a memory 420, a communication module 430, a display 440, and a sensor module 450. In some embodiments, the portable device 400 may omit at least one of the constituent elements or may additionally have other constituent elements.

The processor 410 may include at least one of a central processing unit (CPU), application processor (AP), and communication processor (CP). The processor may execute, for example, calculation or data processing of the control and/or communication of at least one other constituent element of the portable device 400.

The memory 420 may store one or more programs executed by the processor 410 and perform a function for temporary storage of input/output data. The input/output data may include, for example, data for controlling a light emitting unit, light receiving unit, or light splitter of a spectroscopic sensor and biometric data including the degree of hydration of a user.

The communication module 430 may include, for example, wireless communication or wire communication. Wireless communication may include cellular communication that uses at least one of, for example, long-term evolution (LTE), LTE advance (LTE-A), code division multiple access (CDMA), wideband CDMA (WCDMA), universal mobile telecommunications system (UMTS), wireless broadband (WiBro), and global system for mobile communications (GSM). According to an embodiment, wireless communication may include, for example, at least one of wireless fidelity (WiFi), Bluetooth, Bluetooth low energy (BLE), Zigbee, near field communication (NFC), magnetic secure transmission, radio frequency (RF), and body area network (BAN). According to an embodiment, wireless communication may include a global navigation satellite system (GNSS). The GNSS may be, for example, a global positioning system (GPS), global navigation satellite system (Glonass), Beidou navigation satellite system (hereinafter "Beidou") or Galileo, and European global satellite-based navigation system. Hereinafter, in this document, "GPS" may be interchangeably used with "GNSS". Wire communication may include at least one of, for example, a universal serial bus (USB), a high definition multimedia interface (HDMI), recommended standard 232 (RS-232), power line communication, and plain old telephone service (POTS). According to an embodiment of the present invention, the portable device 400 may transmit data measured by at least one sensor included in the sensor module 450 to another electronic device or a server using at least one communication method included in the communication module 430 and receive information (e.g., measurement data of other users) related to measured data from another electronic device or server.

The display 440 may serve as an intermediary for showing an output from the portable device 400 to the user. For example, the display 440 may show a visual output to the user. The visual output may appear in the form of a text, graphic, video, and combinations thereof. According to embodiments of the present invention, the display 440 may display various screens according to an operation of the portable device 400. The various screens may include, for example, a user interface screen for controlling the spectroscopic sensor or a user interface screen for displaying biometric data of the user.

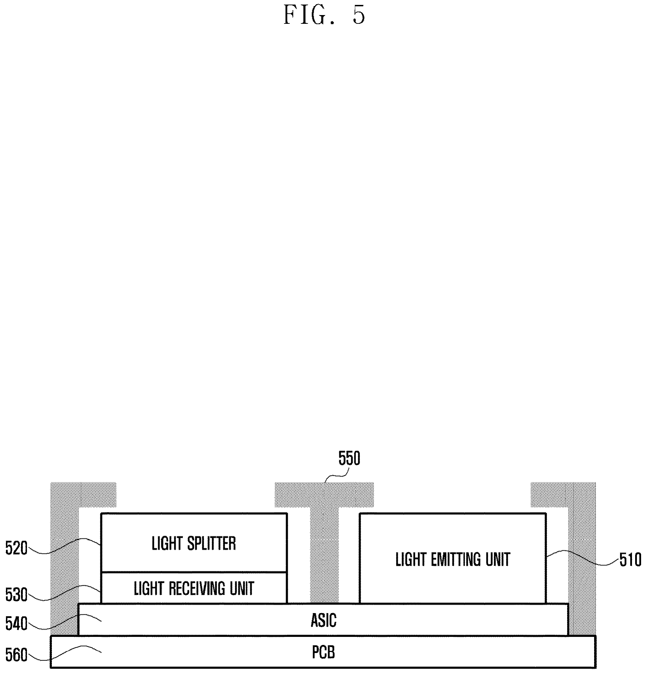

The sensor module 450 may measure, for example, a physical quantity or detect an operation state of the portable device 400 and convert measured or detected information to an electric signal. The sensor module 450 may include, for example, at least one of a spectroscopic sensor 450a and a galvanic skin response (GSR) sensor 450b. The sensor module 450 may further include a control circuit for controlling at least one sensor that belongs therein. In another embodiment, the portable device 400 further includes a processor configured to control the sensor module 450 as a portion of the processor 410 or separately from the processor 410, and when the processor 410 is in a sleep state, the processor may control the sensor module 450. The spectroscopic sensor 450a will be described in detail with reference to FIG. 5.

A portable device according to the present invention includes a housing; a light emitting unit positioned on at least one surface of the housing and configured to radiate light; a light splitter configured to split the light into at least one wavelength band; a light receiving unit positioned on the at least one surface of the housing and configured to receive at least some of the light; and a processor electrically connected to at least one of the light emitting unit, the light splitter, and the light receiving unit, wherein the processor is configured to control the light emitting unit to radiate light to an object, to control the light splitter to split the light, to control the light receiving unit to obtain a spectrum of a first wavelength band based on at least some light scattered and returned among the light radiated to the object, and to determine a first degree of hydration of the object based on the obtained spectrum of the first wavelength band.

The light splitter of the portable device according to various embodiments of the present invention may be positioned at the upper end of the light emitting unit or the light receiving unit.

The portable device according to various embodiments not covered by the present invention may further include a sensor that can measure electrical conductivity, wherein the processor may be configured to determine a second degree of hydration of the object using the sensor.

The processor of the portable device according to the present invention is configured to control the light receiving unit to obtain a spectrum of a second wavelength band based on at least some light scattered and returned among the light radiated to the object and to determine a second degree of hydration of the object based on the obtained spectrum of the second wavelength band.

The portable device according to various embodiments not covered by the present invention may further include a polarizing film configured to pass through light applied from a specific angle, wherein the processor may be configured to determine a second degree of hydration of the object based on light received by passing through the polarizing film.

The processor of the portable device according to the present invention is configured to determine a third degree of hydration of the object based on the determined first degree of hydration and second degree of hydration.

A portable device according to the present invention includes a memory and a display, wherein the processor may be configured to control the memory to collect biometric data including the determined third degree of hydration and to control the display to display a personalized UI based on biometric data collected in the memory.

The portable device according to various embodiments of the present invention may further include at least one sensor, wherein the processor may be configured to control the at least one sensor to detect proximity between the portable device and the object and to control the light emitting unit to radiate light to the object when proximity is detected.

A portable device according to various embodiments of the present invention includes a housing; a light emitting unit positioned on at least one surface of the housing and configured to radiate light of at least two different wavelength bands; a light receiving unit positioned on the at least one surface of the housing and configured to receive at least some of the light of the two different wavelength bands; and a processor electrically connected to at least one of the light emitting unit and the light receiving unit, wherein the processor is configured to control the light emitting unit to radiate light of at least two different wavelength bands to the object, to control the light receiving unit to obtain spectra of the first and second wavelength bands based on light of at least two different wavelength bands radiated from the light emitting unit, and to determine a first degree of hydration and a second degree of hydration of the object based on the obtained spectra of the first and second wavelength bands.

The processor of the portable device according to the present invention is configured to determine a third degree of hydration of the object based on the determined first degree of hydration and second degree of hydration of the object.

According to various embodiments of the present invention, the first degree of hydration includes a degree of hydration of an entire skin, the second degree of hydration includes a degree of hydration of a skin layer, and the third degree of hydration includes a degree of hydration of a dermal layer.

FIG. 5 is a cross-sectional view illustrating a spectroscopic sensor 450a according to various embodiments.

With reference FIG. 5, the spectroscopic sensor 450a may include a light emitting unit 510, light splitter 520, light receiving unit 530, application specific integrated circuit (ASIC) 540, partition wall 550, and printed circuit board (PCB) 560.

The light emitting unit 510 may radiate broadband light including a wavelength band to obtain a spectrum. The light emitting unit 510 may include, for example, a halogen lamp or a light emitting diode (LED) that can output broadband light including a near-infrared band. In some embodiments, the halogen lamp or a light source 510 such as an LED may be configured to radiate light including a near-infrared band having wavelengths between 500 nm and 2000 nm. Broadband light radiated from the light emitting unit 510 does not necessarily include a near infrared ray band and the difference of absorption spectra according to an amount of moisture may be determined even in other wavelength bands.

The light splitter 520 may be a device for splitting light generated by the light emitting unit 510 on a wavelength basis before reaching the light receiving unit 530 through various paths. For example, the light splitter 520 may include diffraction grating, a Fabry-Perot Interferometer (FPI), a linear variable filter (LVF), and digital light processing (DLP) as well as a general filter such as a low pass filter, high pass filter, bandpass filter, and notch filter. Further, the light splitter 520 may include a reflection plate and a lens for adjusting a path and form of light. With reference to FIG. 5, the light splitter 520 may be positioned at the upper end of the light receiving unit 530. However, the light splitter 520 is not limited thereto and, for example, the light splitter 520 may be positioned at the upper end of the light emitting unit 510. In another embodiment, when a wavelength of light radiated from the light emitting unit 510 is a short wavelength (e.g., LED), the light splitter 520 may be omitted because spectroscopy is not required. In another embodiment not covered by the present invention, when at least two light emitting units radiate light of at least two different wavelength bands, the light splitter 520 may be omitted because spectroscopy is not required.

The light receiving unit 530 measures the intensity of split light by changing an amount of applied photons to a current. For example, the light receiving unit 530 may include a photodiode and a pyroelectric detector.

The ASIC 540 may include an LED driver IC for controlling the light source 510, an oscilloscope (OSC) for adjusting a microcontroller unit (MCU) and a light receiving unit 530, and an analog circuit chip set (e.g., an analog front-end (AFE)).

The partition wall 550 may be a structure for preventing light generated in the light source 510 from being directly introduced into the light receiving unit 530 when the light is returned through various paths.

The printed circuit board (PCB) 560 may be a board for controlling the light source 510 to generate light and for analyzing a spectrum obtained based on a value of a current measured by the light receiving unit 530.

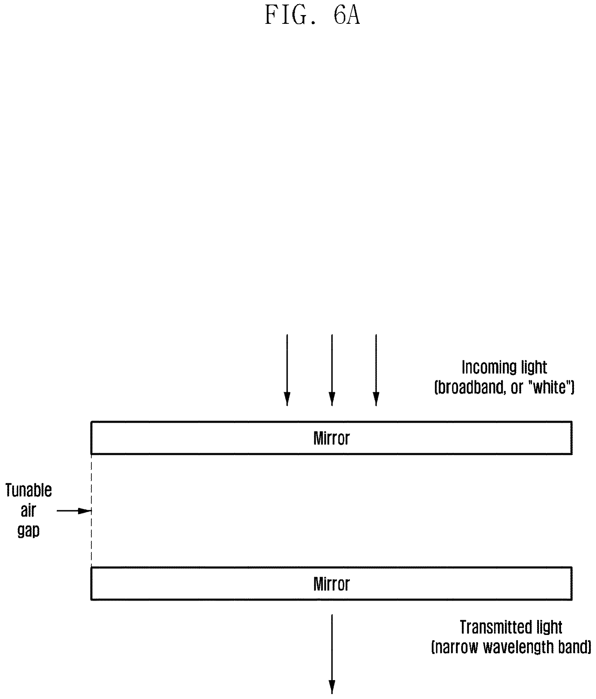

FIG. 6A is a cross-sectional view illustrating a Fabry-Perot Interferometer (FPI) filter according to an embodiment of a light splitter.

With reference to FIG. 6A, the FPI filter may include two mirrors and an actuator for adjusting a distance between the mirrors. For example, when multiple wavelengths are applied to the FPI filter, the FPI filter generates multiple interference phenomena in a tunable air gap to transmit only specific wavelengths and to reflect other wavelengths, thereby selecting only desired data. The FPI filter is to use the principle that a transmitted wavelength band varies according to a distance between the two mirrors.

The distance between the mirrors may be adjusted by an actuator. The FPI filter is a time-division type light splitter that obtains information of one wavelength band in a short time of several ms and that senses the other wavelength band while changing the distance.

According to an embodiment, the FPI filter may be fixed at one mirror distance and sense a temporal change of the same wavelength band. For example, a mirror distance for measuring heartbeats may be fixed and a temporal change may be sensed.

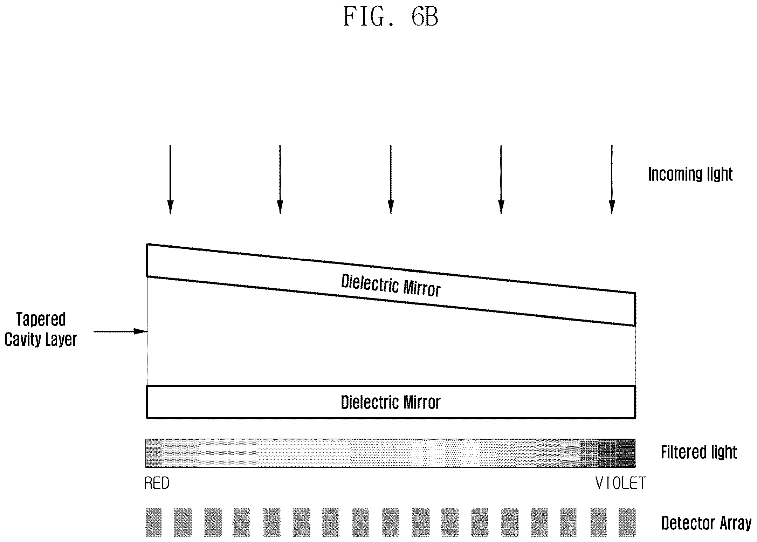

FIG. 6B is a cross-sectional view illustrating a linear variable (LV) filter according to an embodiment of a light splitter.

With reference to FIG. 6B, the LV filter physically differently fixes a distance between two mirrors and senses each wavelength band in the light receiving unit matched to each distance. Therefore, the LV filter may be a position-division type light splitter unlike the FPI filter. However, in a single light receiving unit, the LV filter may sense and use a temporal change of the same wavelength band.

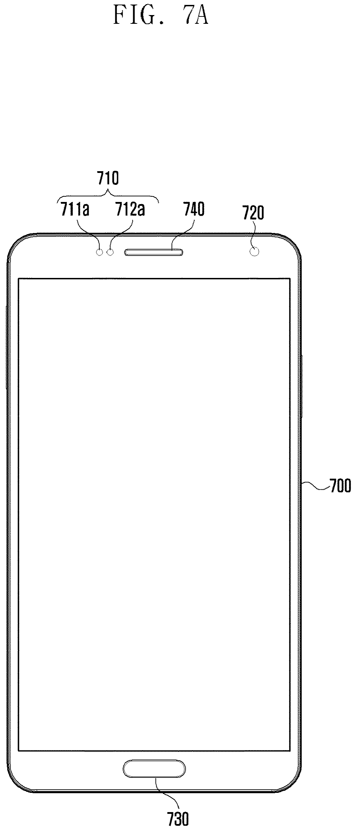

FIGS. 7A to 7C are diagrams illustrating a portable device including a spectroscopic sensor according to an embodiment.

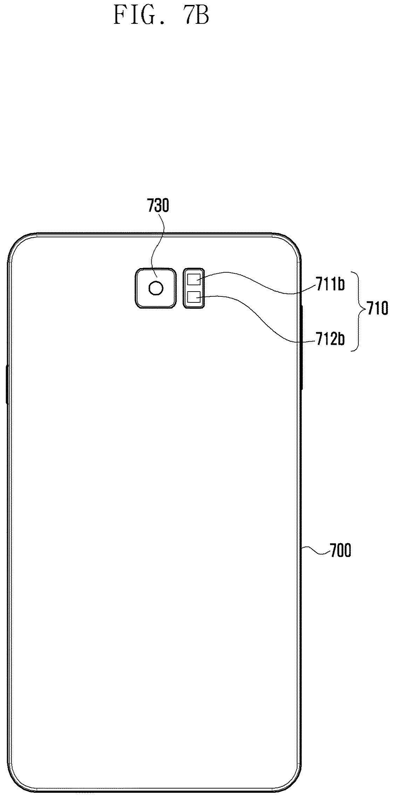

With reference to FIGS. 7A and 7B, a portable device 700 may include various electronic components and a housing for protecting them. The housing may include a first surface, a second surface facing in an opposite direction of the first surface, and a side surface enclosing at least a portion of a space formed between the first surface and the second surface.

With reference to FIG. 7A, a spectroscopic sensor 710 may be disposed in at least a partial area of the first surface of the housing. In at least a partial area of the first surface of the housing, a display may be positioned. In order to perform a function of displaying an image and an input function by a user's touch, the display may include a touch screen or a touch panel. In at least a partial area of the first surface in which the display is not positioned, other constituent elements (e.g., a speaker, sensor, spectroscopic sensor 710, front camera 720, key button 730, or receiver 740) may be disposed. In particular, the spectroscopic sensor 710 may be disposed such that both a light source 711a and a light receiving unit 712a of the spectroscopic sensor 710 are positioned in at least a partial area of the first surface of the housing.

With reference to FIG. 7B, the spectroscopic sensor 710 may be disposed in at least a portion of the second surface of the housing. For example, in at least a partial area of the second surface of the housing facing in an opposite direction of the first surface, a spectroscopic sensor 710, a rear surface camera 730, a sensor, or a flash may be positioned. In particular, the spectroscopic sensor 710 may be disposed such that both a light source 711b and the light receiving unit 712b of the spectroscopic sensor 710 are positioned in at least a partial area of the second surface of the housing.

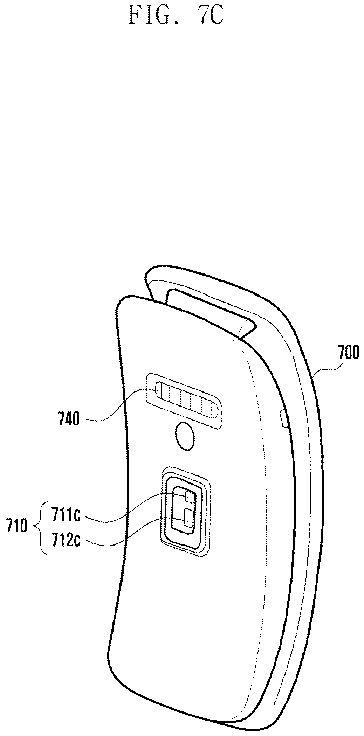

FIG. 7C illustrates an embodiment in which the portable device 700 is a wearable device (e.g., a smart watch, a smart band). A light source 711c, light receiving unit 712c, or charging terminal 740 of the spectroscopic sensor 710 may be disposed in at least a partial area of one surface of the wearable device. In this case, the spectroscopic sensor 710 may be configured to be positioned at one surface in which the wearable device comes at least in contact with a wearer's skin.

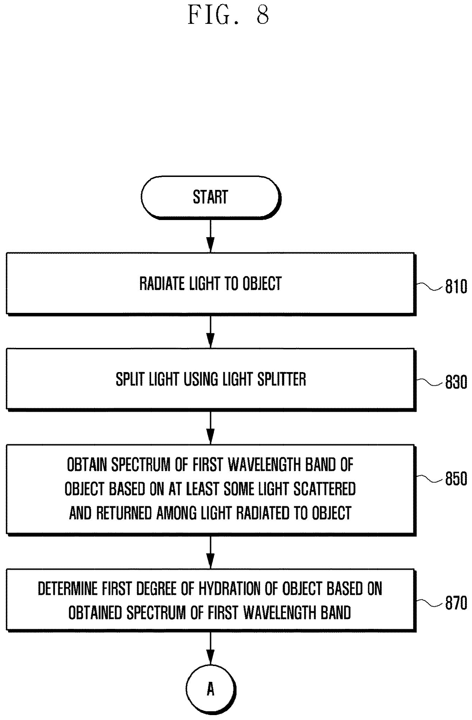

FIG. 8 is a flowchart illustrating a method of measuring the degree of hydration of an object according to various embodiments.

With reference to FIG. 8, at operation 810, the processor 410 included in the portable device 400 may control the light emitting unit 510 to radiate light to an object.

According to various embodiments, measurement of the degree of hydration of an object using the portable device 400 may be started by radiating broadband light to the object. Further, measurement of the degree of hydration of the object may be started in various methods. For example, upon receiving a user's manual input while entering a particular application, the processor 410 may start measurement of the degree of hydration of an object. In some embodiments, when a particular event (e.g., image photographing) occurs, it may be preset to measure the degree of skin hydration before/after the particular event. In another embodiment, biometric information of the user may be measured, and when it is determined that the user is in an emergency situation, the degree of skin hydration may be measured. In another embodiment, the period may be preset and the degree of hydration of the object may be measured according to the preset period. In another embodiment, when it is detected that the portable device 400 approaches the object, the degree of hydration of the object may be measured. For example, the portable device 400 may detect proximity between a spectroscopic sensor (e.g., a light emitting unit 510 or a light receiving unit 530 of the spectroscopic sensor) of the portable device 400 and an object (e.g., human skin), and when the portable device 400 determines proximity between a spectroscopic sensor (e.g., a light emitting unit 510 or a light receiving unit 530 of a spectroscopic sensor) of the portable device 400 and an object (e.g., human skin) as a detection result, measurement of the degree of hydration of the object may be started. That is, the portable device 400 may include at least one sensor for determining proximity between the spectroscopic sensor and the object, and when the at least one sensor detects proximity between the portable device 400 and the object, the processor 410 may control the at least one sensor to start hydration measurement.

According to various embodiments, when it is determined that the start of measurement of the degree of hydration of the object by various methods has been entered, the processor 410 may apply a current to the spectroscopic sensor 450a to activate the spectroscopic sensor 450a. That is, the processor 410 may apply a current to the light emitting unit 510 of the spectroscopic sensor 450a to activate the light emitting unit 510. In order to stably activate broadband light radiated to the light emitting unit 510, the processor 410 may perform some standby operations. For example, the processor 410 may activate the light emitting unit 510 and control the light emitting unit 510 not to radiate light for about one second, and control the light emitting unit 510 to radiate broadband light after about 1 second has elapsed.

At operation 830, the processor 410 included in the portable device 400 controls the light splitter 520 to split the light.

According to various embodiments, the light splitter 520 may split light before light radiated from the light emitting unit 510 reaches the object or may split light before light scattered by and returned to the object reaches the light receiving unit 530.

In order to obtain a desired spectrum from light radiated from the light emitting unit 510, the spectroscopic sensor 450a includes a light splitter 520. In order to obtain a desired spectrum, the light splitter 520 may include a general filter such as a low pass filter, high pass filter, band pass filter, and notch filter and include diffraction grating, Fabry-Perot interferometer (FPI), linear variable filter (LVF), and digital light processing (DLP). The light splitter 520 may split obtained light to include light of at least a first wavelength band.

At operation 850, the processor 410 included in the portable device 400 controls the light receiving unit 530 to obtain a spectrum of the first wavelength band of the object based on light scattered and returned among light radiated to the object.

According to the present invention, the light receiving unit 530 receives light scattered and returned among light radiated to the object.

Light radiated to an object (e.g., skin) may be scattered in various directions, especially it may be inelastically scattered by colliding with atoms or molecules in an object. This scattering may be scattering absorbed and emitted by an atom or molecule rather than being simply reflected from a surface of an atom or molecule. Therefore, the scattered light may have a longer wavelength than that of radiated light. For example, light radiated from the light emitting unit 510 and light scattered from the object may have a wavelength difference in a range of about 200 nm or less. In other words, because light radiated to the object collides with a molecular structure in the object and is absorbed into the molecular structure and then again released, the light may come out of the object in the form of scattered light in which a wavelength is converted. For example, light radiated to the skin may be absorbed by blood plasma in the skin and then released out of the skin in the form of scattered light in which a wavelength is converted.

In order to measure an intensity of light split into a first wavelength band, the light receiving unit 530 may change and measure an amount of applied photons to a current. For example, when a photodiode is used as the light receiving unit 530, light is applied to a depletion region of the photodiode to generate electron-hole pairs, and the light receiving unit 530 extracts the generated electrons (or holes), thereby converting an optical signal to an electrical signal. In this case, a generated amount of electron-hole pairs may vary according to an intensity of absorbed light. As the intensity of absorbed light increases, a generated amount of electron-hole pairs may increase. That is, in order to obtain a spectrum of the first wavelength band, the light receiving unit 530 may output received light to an electrical signal and enable the portable device 400 to analyze the electrical signal to obtain a spectrum of the first wavelength band.

According to various embodiments, an operation of obtaining a spectrum in order to more accurately determine the degree of hydration of an object may be repeatedly performed. For example, an operation of obtaining a spectrum of scattered light reflected from the skin may be performed 10 times or more, and an average value of spectra obtained by performing the operation 10 times or more may be calculated.

At operation 870, the processor 410 included in the portable device 400 determines a first degree of hydration of the object based on the obtained spectrum of the first wavelength band.

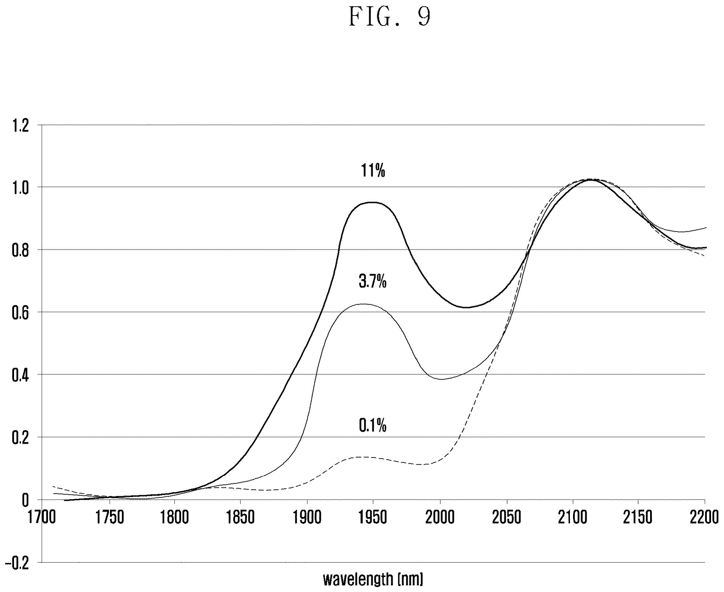

According to various embodiments, the degree of hydration of an object may be calculated based on a moisture content prediction model such as partial least squares (PLS) obtained from an experiment. For example, an absorption amount of the obtained spectrum in a specific wavelength band may be determined, and the degree of hydration of the object may be determined based on the determined absorption amount. A method of determining the degree of hydration of the skin based on the PLS prediction model will be described in detail with reference to FIG. 9.

According to the present invention, the first degree of hydration means the degree of hydration of an entire skin, for example, the degree of hydration included in all tissues such as the epidermis and dermis constituting the skin.

FIG. 9 is a graph illustrating a prediction model of an absorption spectrum according to the degree of hydration of an object according to various embodiments.

An absorption spectrum may vary according to the degree of hydration of the object. Therefore, a moisture content prediction model of partial least squares (PLS) as in FIG. 9 may be generated based on an absorption spectrum of many samples, thereby determining the degree of hydration. With reference to FIG. 9, an amount of absorption may be determined in a band having a wavelength of 1950 nm and the degree of hydration of the skin may be determined based on the amount of absorption. This is because the difference in an amount of absorption according to the degree of skin hydration in a band having a wavelength of 1950 nm is relatively clear.

For example, when an absorption amount measured in the band of 1950 nm is not 0.2, it may be determined that a moisture content is less than about 0.1%. For another example, when an absorption amount measured in a band having a wavelength of 1950 nm is about 0.6, a moisture content may be determined to about 3.7%. In another example, when an absorption amount measured in a band having a wavelength of 1950 nm is about 1.0, a moisture content may be determined to about 11%.

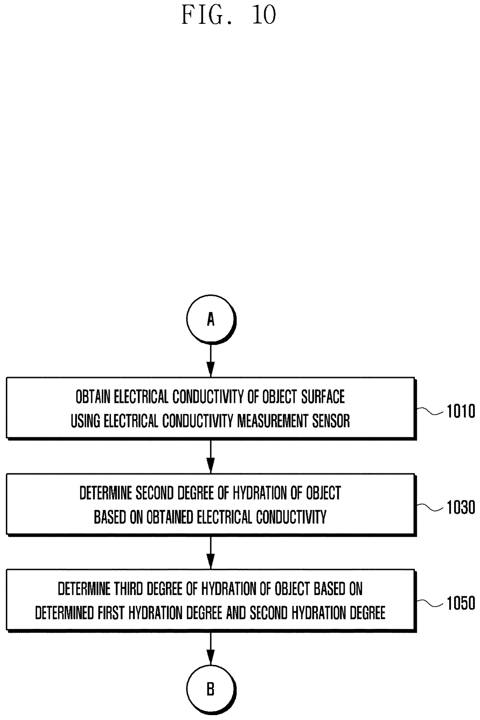

FIG. 10 is a flowchart illustrating a method of determining a second degree of hydration of an object using electrical conductivity of an object surface according to various embodiments not covered by the present invention.

Operations shown in FIG. 10 may operate sequentially after operation 870 of FIG. 8.

At operation 1010, the processor 410 included in the portable device 400 may control at least one electrical conductivity measurement sensor to obtain electrical conductivity of an object surface.

The electrical conductivity measurement sensor may include a capacitance based sensor (e.g., a galvanic skin response (GSR) sensor). The GSR sensor may measure electrical conductivity of a user's skin to obtain skin electrical resistance (or electrical conductivity). Because the skin contains a large amount of electrolytes such as salts and amino acids in an outer layer, the GSR sensor uses the principle that a surface of the skin has an electro conductive property in the presence of moisture. That is, the processor 410 may use an electric conductivity measurement sensor to flow an alternating current of a predetermined frequency to a surface (e.g., a skin layer) of an object and obtain electric conductivity, which is an inverse number of a resistor constituting impedance.

At operation 1030, the processor 410 included in the portable device 400 may determine a second degree of hydration of the object based on the obtained electrical conductivity.

The memory 420 included in the portable device 400 may store a correlation between electrical conductivity and the degree of hydration of the object. For example, when using a correlation table between skin electrical resistance previously stored in the memory 420 and the degree of hydration of the skin layer, a second degree of hydration of the skin may be determined based on a skin electrical resistance value obtained using the GSR. A method of using a correlation table between skin electrical resistance and the degree of hydration of the skin layer is obvious to a person skilled in the art; thus, a detailed description thereof will be omitted.

The second degree of hydration means the degree of hydration of a surface of the object and mean, for example, the degree of hydration of a skin layer in the skin.

At operation 1050, the processor 410 included in the portable device 400 may determine a third degree of hydration of the object based on the determined first hydration degree and second hydration degree.

The first degree of hydration determined according to the flowchart of FIG. 8 may include noise of a surface of the object. For example, the first degree of hydration may be determined by including contamination of a skin epidermal layer and effects of external temperature or humidity. Therefore, in order to determine the degree of hydration of the object itself, it is necessary to correct noise of a surface of the object.

In particular, because the degree of hydration of the skin means the degree of hydration contained in the blood, a third degree of hydration of the object may be determined based on the determined first degree of hydration and second degree of hydration. That is, by subtracting the second degree of hydration from the first degree of hydration, the third degree of hydration, for example the degree of hydration of a dermal layer, may be determined.

Therefore, the third degree of hydration may mean the degree of hydration of the object itself and represent the degree of hydration of a dermal layer of the skin in which the degree of hydration of an outer layer of the skin is corrected in the degree of hydration of the entire skin.

When only the degree of hydration of an outer layer in the skin is required, operation 1050 may be omitted. For example, in the case of a service for measuring skin health of the user, a service for measuring the degree of the user's sweating according to exercise, and a service for calculating the degree of the user's skin response to ultraviolet rays, only the degree of hydration of an outer layer in the skin may be required. Accordingly, when the above-described services are provided, operation 1050 may be omitted.

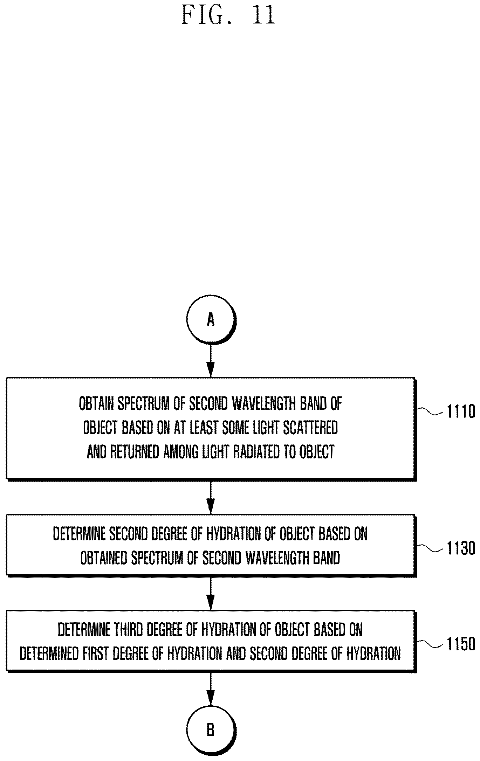

FIG. 11 is a flowchart illustrating a method of determining a second degree of hydration of an object using characteristics of different wavelength bands according to various embodiments.

Operations of FIG. 11 may operate sequentially after operation 870 of FIG. 8.

At operation 1110, the processor 410 included in the portable device 400 controls the light receiving unit 530 to obtain a spectrum of a second wavelength band of the object based on scattered and returned light among light radiated to the object at operation 830.

According to various embodiments, the second wavelength band may be a band having characteristics that may not reach the interior of the object (e.g., a dermal layer in the skin). Light radiated at operation 810 may be radiation of light including a first wavelength band and a second wavelength band. Thereafter, the light splitter may split light scattered by the object and returned to the light splitter into a first wavelength band and a second wavelength band. Therefore, the second wavelength band that does not pass through the object may have only information about the degree of hydration of a surface of the object. For example, the light emitting unit may radiate light including at least a band of 970 nm and/or 1450 nm. The 970 nm or 1450 nm may mean a wavelength band used for measuring the degree of hydration of at least the entire skin. In some embodiments, the light emitting unit may radiate light including a wavelength band of at least 1940 nm to an object. The light emitting unit is to use the point that light of a band having a wavelength of about 1940 nm radiated to the skin may not reach a dermis layer but the light of the band may only reach a relatively thin skin layer.

When light including a wavelength band of about 1940 nm is radiated to the skin, the light including a wavelength band of about 1940 nm may be absorbed only into blood plasma of the skin layer, and a spectrum of light absorbed into the blood plasma may be converted and released. Thus, the portable device may control the spectroscopic sensor to split light including the 1940 nm wavelength band and control the light receiving unit to obtain a spectrum of a second wavelength band of the object based on the light split by the light splitter.

At operation 1130, the processor 410 included in the portable device 400 determines a second degree of hydration of the object based on the obtained spectrum of the second wavelength band.

According to various embodiments, the second degree of hydration of the object may be calculated based on a moisture content prediction model such as PLS, obtained as a result of an experiment, as in operation 870. A method of determining the degree of hydration of the skin based on the PLS prediction model has been described in detail with reference to FIG. 9; thus, a detailed description thereof will be omitted.

At operation 1150, the processor 410 included in the portable device 400 determines a third degree of hydration of the object based on the determined first degree of hydration and second degree of hydration.

Similar to operation 1050, the first degree of hydration determined according to the flowchart of FIG. 4 may include noise of a surface of an object. Therefore, in order to determine the degree of hydration of the object itself, it is necessary to correct noise of the surface of the object. That is, by subtracting the second degree of hydration from the first degree of hydration, the third degree of hydration, for example the degree of hydration of the dermal layer, may be determined. Operation 1150 is substantially the same as operation 1050; thus, a detailed description thereof will be omitted.

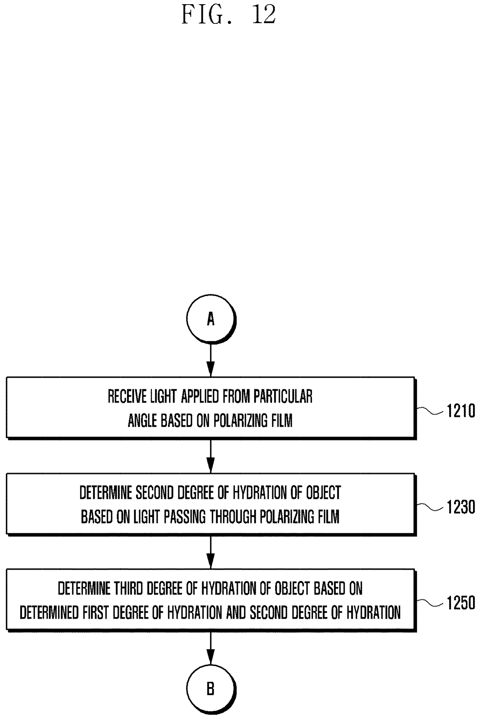

FIG. 12 is a flowchart illustrating a method of determining a second degree of hydration of an object using a polarizing film according to various embodiments not covered by the present invention.

Operations of FIG. 12 may operate sequentially after operation 870 of FIG. **8**.

At operation 1210, the processor 410 included in the portable device 400 may receive light applied from a particular angle based on a polarizing film.

The polarizing film may be configured to pass through only light scattered from a surface of the object. For example, the polarizing film may be configured to pass through only light scattered from the skin layer in consideration of an angle of light scattered from the skin layer and applied to the spectroscopic sensor.

At operation 1230, the processor 410 included in the portable device 400 may determine a second degree of hydration of the object based on light passing through the polarizing film.

Because the light passing through the polarizing film has only information on the degree of hydration of an object surface, the second degree of hydration of the object may be determined based on the light passing through the polarizing film.

The second degree of hydration of the object may be calculated based on a moisture content prediction model, such as PLS, obtained as a result of an experiment, as in operation 870. A method of determining the degree of hydration of the skin based on the PLS prediction model has been described in detail with reference to FIG. 9; thus, a detailed description thereof will be omitted.

At operation 1250, the processor 410 included in the portable device 400 may determine a third degree of hydration of the object based on the determined first degree of hydration and second degree of hydration.

Similar to the operation 1050, the first degree of hydration determined according to the flowchart of FIG. 8 may include noise of a surface of the object. Therefore, in order to determine the degree of hydration of the object itself, it is necessary to correct noise of the surface of the object. That is, by subtracting the second degree of hydration from the first degree of hydration, the third degree of hydration, for example the degree of hydration of the dermal layer, may be determined. Operation 1250 is substantially the same as operation 1050; thus, a detailed description thereof will be omitted.

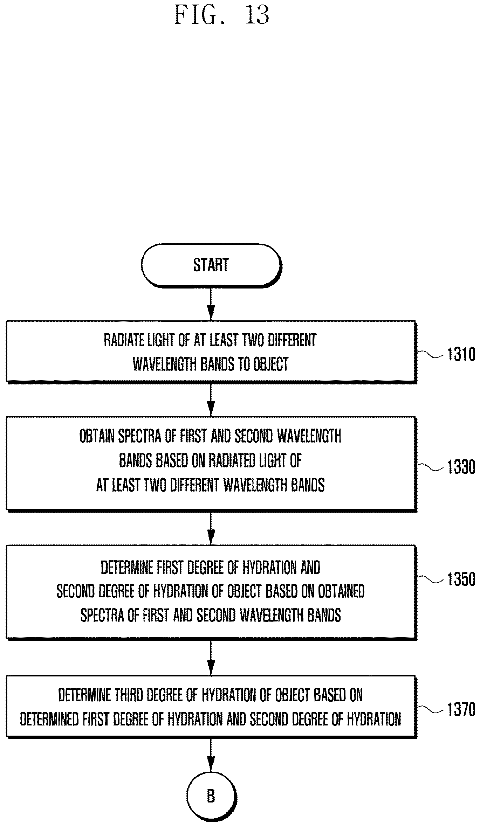

FIG. 13 is a flowchart illustrating a method of determining a third degree of hydration in a portable device 400 excluding a light splitter according to various embodiments not covered by the present invention.

With reference to FIG. 13, at operation 1310, the processor 410 included in the portable device 400 may control the light emitting unit 510 to radiate light of at least two different wavelength bands to the object.

There may be one light emitting unit that radiates light of at least two different wavelength bands; and, in some embodiments, a first light emitting unit that radiates light of a specific wavelength band and a second light emitting unit that radiates light of a wavelength band different from that of a specific wavelength band may be separated. Therefore, the portable device may include at least one light emitting unit that radiates light of at least two different wavelength bands.

At operation 1330, the portable device may control the light receiving unit 530 to obtain spectra of the first and second wavelength bands based on the light of at least two different wavelength bands radiated from the light emitting unit.

A single light receiving unit may receive all of the light of at least two different wavelength bands radiated from the light emitting unit and obtain a spectrum of the first wavelength band and a spectrum of the second wavelength band based on each received light. Alternatively, the light receiving unit may be configured to be divided into a first light receiving unit that receives light of a specific wavelength band and a second light receiving unit that receives light of a wavelength band different from that of the specific wavelength band to enable each light receiving unit to obtain a spectrum of the first wavelength band and a spectrum of the second wavelength band. Accordingly, the portable device may include at least one light receiving unit that receives at least some light scattered and returned among light radiated to the object.

At operation 1350, the processor 410 included in the portable device 400 may determine a first degree of hydration and a second degree of hydration of the object based on the obtained spectra of the first and second wavelength bands.

A method of determining the degree of hydration of an object based on the obtained wavelength band may be determined based on a moisture content prediction model, such as PLS, obtained from an experiment result, as in operation 870. A method of determining a hydration degree of the skin based on the PLS prediction model has been described in detail with reference to FIG. 9; thus, a detailed description thereof will be omitted

At operation 1370, the processor 410 included in the portable device 400 may determine a third degree of hydration of the object based on the determined first degree of hydration and second degree of hydration.

The determined first degree of hydration may include noise of a surface of the object. Therefore, in order to determine the degree of hydration of the object itself, it is necessary to correct noise of the surface of the object. That is, by subtracting the second degree of hydration from the first degree of hydration, the third degree of hydration, for example the degree of hydration of the dermal layer, may be determined. Operation 1370 is substantially the same as operation 1050; thus, a detailed description thereof will be omitted.

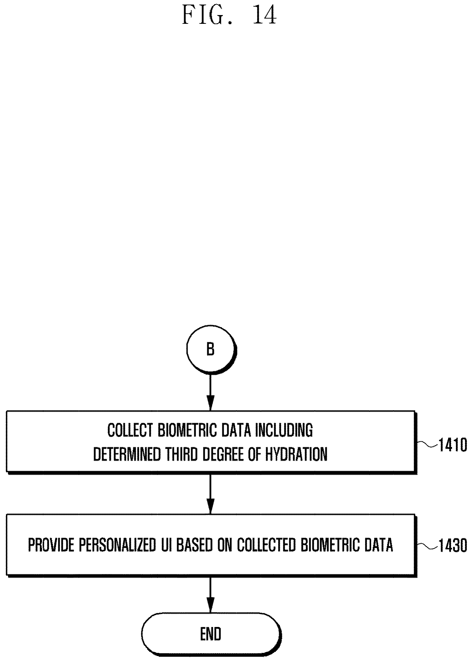

FIG. 14 is a flowchart illustrating a method of providing a personalized UI using a determined third degree of hydration according to the present invention.

Operations shown in FIG. 14 may be operated sequentially after determining the third degree of hydration.

At operation 1410, the processor 410 included in the portable device 400 collects biometric data including the determined third degree of hydration.

According to various embodiments, the processor 410 may collect biometric data including a third degree of hydration determined in real-time data and/or cumulative data format. For example, the real-time data may include a numerical value indicating how much moisture is contained in blood of a current user. The cumulative data may include a numerical value indicating how much moisture the user has consumed for a specified period of time.

At operation 1430, the processor 410 included in the portable device 400 provides a personalized UI based on the collected biometric data.

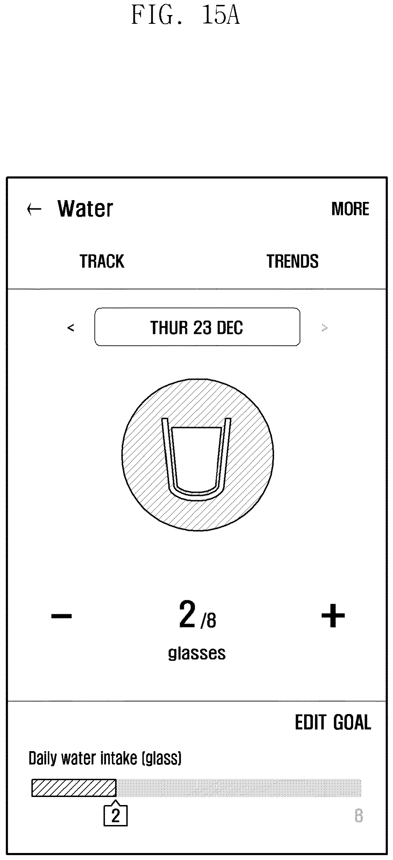

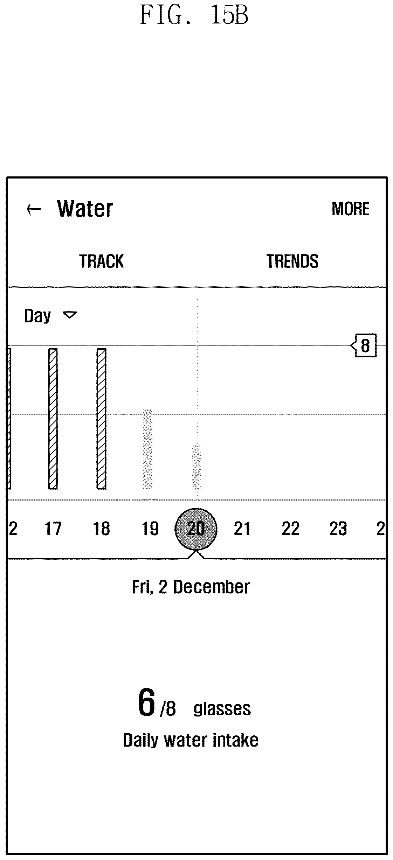

FIGS. 15A and 15B are diagrams illustrating a personalized UI according to various embodiments.

FIG. 15A illustrates a UI for displaying an amount of water consumed by the user in a cup so as to be intuitively recognized. For example, when the increased degree of hydration in blood of a user at a specified segment is known, it may be calculated inversely to determine how many cups of water the user has drunk.

With reference to FIG. 15B, an intake water amount may be displayed in a trend format according to hour, day, month, and year.

According to various embodiments, the collected biometric data may be provided to an application or an external service provider. In particular, when using a spectroscopic sensor without having to manually input the user's own moisture information, the moisture information may automatically be provided to an application or an external service provider. When using the provided biometric data, the measured moisture information may be provided to a provider providing services such as health, fitness, and diet.

A method of measuring the degree of hydration of an object using a portable device according to the present invention includes radiating light to an object, splitting the light, obtaining a spectrum of a first wavelength band based on at least some light scattered and returned among the light radiated to the object, and determining a first degree of hydration of the object based on the obtained spectrum of the first wavelength band.

A method of measuring the degree of hydration of an object using a portable device according to the present invention further includes obtaining a spectrum of a second wavelength band of the object based on at least some light scattered and returned among light radiated to the object, and determining a second degree of hydration of the object based on the obtained spectrum of the second wavelength band.

A method of measuring the degree of hydration of an object using a portable device according to the present invention further includes determining a third degree of hydration of the object based on the determined first degree of hydration and second degree of hydration.

Although a portable device and a method of measuring skin hydration using the same according to embodiments of the present invention have been described in detail hereinabove, it should be clearly understood that many variations and modifications of the basic inventive concepts herein described that may appear to those skilled in the art will still fall within the scope of the embodiments of the present invention as defined in the appended claims.

## Claims

1. A portable device (101, 201, 400, 700), comprising:
a housing;
a memory;
a light emitting unit (510) positioned on at least one surface of the housing and configured to radiate light;
a light splitter (520) configured to split the light into at least one wavelength band;
a light receiving unit (530) positioned on the at least one surface of the housing and configured to receive at least some of the light;
a display (440); and
a processor (410) electrically connected to at least one of the light emitting unit, the light splitter, the light receiving unit, and the display,
wherein the processor (410) is configured to:
control (810) the light emitting unit to radiate light to a skin of a user,
control (830) the light splitter to split the light,
control (850) the light receiving unit to obtain a first spectrum of a first wavelength band based on at least some light scattered and returned among the light radiated to the skin,
determine (870) a first degree of hydration associated with the entire skin based on the obtained first spectrum,
control (1110) the light receiving unit to obtain a second spectrum of a second wavelength band based on at least some light scattered and returned among the light radiated to the skin,
determine (1130) a second degree of hydration associated with a surface of the skin based on the obtained second spectrum,
determine (1150) a third degree of hydration associated with a dermis of the skin by subtracting the second degree of hydration from the first degree of hydration,
collect (1410) biometric data including the third degree of hydration, and
control (1430) the display to display a personalized user interface (UI) to the user based on the collected biometric data.

2. The portable device of claim 1, wherein the processor (410) is configured to:
identify an amount of water consumed by a user based on the collected biometric data, and
control the display to display the amount of water consumed by the user in the personalized UI.

3. The portable device of claim 1, wherein the light splitter is positioned at the upper end of the light emitting unit or the light receiving unit.

4. The portable device of claim 1,
wherein the processor is configured to control the memory to collect the biometric data comprising the determined third degree of hydration and to control the display to display the personalized user interface (UI) to the user based on the biometric data collected in the memory.

5. The portable device of any preceding claim, wherein the portable device is configured to one or more of determine at least the first degree of hydration:
when a user's manual input is received when entering a particular application of the portable device;
when a particular event occurs;
when the user of the portable device is determined, from biometric information of the user, to be in an emergency situation;
according to a preset period; and
when the portable device is determined to approach the skin within a predetermined proximity.

6. The portable device of any preceding claim, wherein the portable device is configured to collect biometric data including the third degree of hydration, in one or more of:
real-time data format, or
cumulative data format

7. The portable device of claim 6, wherein one or more of:
the real-time data comprises a numerical value indicating how much moisture is contained in blood of a current user of the portable device; and
the cumulative data includes a numerical value indicating how much moisture the user of the portable device has consumed for a specified period of time.

8. The portable device of any of claims 4-7, wherein the collected biometric data is provided to an application or an external service provider.

9. A method of measuring the degree of hydration of a skin of a user using a portable device according to claim 1, performed by the portable device processor, the method comprising:
controlling the light emitting unit to radiate light to the skin;
controlling the light splitter to split the light;
controlling (850) the light receiving unit to obtain a first spectrum of a first wavelength band based on at least some light scattered and returned among the light radiated to the skin;
determining a first degree of hydration associated with the entire skin based on the obtained first spectrum;
controlling (1110) the light receiving unit to obtain a second spectrum of a second wavelength band based on at least some light scattered and returned among the light radiated to the skin;
determining a second degree of hydration associated with a surface of the skin based on the obtained second spectrum;
determining a third degree of hydration associated with a dermis of the skin by subtracting the second degree of hydration from the first degree of hydration;
collecting biometric data including the third degree of hydration; and
controlling the display to display providing a personalized user interface (UI) on the display based on the collected biometric data.

10. A computer program comprising instructions configured to, when run on the processor of the device of claim 1, cause said device to perform the method of claim 9.

## Patentansprüche

1. Tragbare Vorrichtung (101, 201, 400, 700), umfassend:
ein Gehäuse;
einen Speicher;
eine Licht emittierende Einheit (510), die auf mindestens einer Oberfläche des Gehäuses positioniert ist und dazu konfiguriert ist, Licht abzustrahlen;
einen Lichtteiler (520), der dazu konfiguriert ist, das Licht in mindestens ein Wellenlängenband zu teilen;
eine Licht empfangende Einheit (530), die auf der mindestens einen Oberfläche des Gehäuses positioniert ist und dazu konfiguriert ist, mindestens einen Teil des Lichts zu empfangen;
eine Anzeige (440) und
einen Prozessor (410), der mit mindestens einer bzw. einem von der Licht emittierenden Einheit, dem Lichtteiler, der Licht empfangenden Einheit und der Anzeige elektrisch verbunden ist,
wobei der Prozessor (410) konfiguriert ist zum:
Steuern (810) der Licht emittierenden Einheit zum Abstrahlen von Licht auf eine Haut eines Benutzers,
Steuern (830) des Lichtteilers zum Teilen des Lichts,
Steuern (850) der Licht empfangenden Einheit zum Beziehen eines ersten Spektrums eines ersten Wellenlängenbands auf der Basis von mindestens einem Teil des Lichts, das gestreut und zurückgestrahlt wird, von dem Licht, das auf die Haut abgestrahlt wird,
Bestimmen (870) eines ersten Hydratationsgrads, der mit der gesamten Haut assoziiert ist, auf der Basis des bezogenen ersten Spektrums,
Steuern (1110) der Licht empfangenden Einheit zum Beziehen eines zweiten Spektrums eines zweiten Wellenlängenbands auf der Basis von mindestens einem Teil des Lichts, das gestreut und zurückgestrahlt wird, von dem Licht, das auf die Haut abgestrahlt wird,
Bestimmen (1130) eines zweiten Hydratationsgrads, der mit einer Oberfläche der Haut assoziiert ist, auf der Basis des bezogenen zweiten Spektrums,
Bestimmen (1150) eines dritten Hydratationsgrads, der mit einer Dermis der Haut assoziiert ist, durch Subtrahieren des zweiten Hydratationsgrads von dem ersten Hydratationsgrad,
Sammeln (1410) von biometrischen Daten, die den dritten Hydratationsgrad beinhalten, und
Steuern (1430) der Anzeige zum Anzeigen einer personalisierten Benutzeroberfläche (UI) für den Benutzer auf der Basis der gesammelten biometrischen Daten.

2. Tragbare Vorrichtung nach Anspruch 1, wobei der Prozessor (410) konfiguriert ist zum:
Identifizieren einer von einem Benutzer konsumierten Wassermenge auf der Basis der gesammelten biometrischen Daten und
Steuern der Anzeige zum Anzeigen der von dem Benutzer konsumierten Wassermenge in der personalisierten UI.

3. Tragbare Vorrichtung nach Anspruch 1, wobei der Lichtteiler am oberen Ende der Licht emittierenden Einheit oder der Licht empfangenden Einheit positioniert ist.

4. Tragbare Vorrichtung nach Anspruch 1,
wobei der Prozessor dazu konfiguriert ist, den Speicher zum Sammeln der biometrischen Daten, die den bestimmten dritten Hydratationsgrad umfassen, zu steuern und die Anzeige zum Anzeigen der personalisierten Benutzeroberfläche (UI) für den Benutzer auf der Basis der in dem Speicher gesammelten biometrischen Daten zu steuern.

5. Tragbare Vorrichtung nach einem vorhergehenden Anspruch, wobei die tragbare Vorrichtung zu einem oder mehreren eines Bestimmens von mindestens dem ersten Hydratationsgrad konfiguriert ist:
wenn eine manuelle Eingabe eines Benutzers empfangen wird, wenn eine bestimmte Anwendung der tragbaren Vorrichtung eingegeben wird;
wenn ein bestimmtes Ereignis eintritt;
wenn von dem Benutzer der tragbaren Vorrichtung aus biometrischen Informationen des Benutzers bestimmt wird, dass er sich in einer Notfallsituation befindet;
gemäß einer voreingestellten Periode und
wenn von der tragbaren Vorrichtung bestimmt wird, dass sie sich der Haut innerhalb einer vorbestimmten Nähe nähert.

6. Tragbare Vorrichtung nach einem vorhergehenden Anspruch, wobei die tragbare Vorrichtung dazu konfiguriert ist, biometrische Daten, die den dritten Hydratationsgrad beinhalten, in einem oder mehreren der folgenden zu sammeln:
Echtzeit-Datenformat oder
kumulatives Datenformat.

7. Tragbare Vorrichtung nach Anspruch 6, wobei eine oder mehrere von:
den Echtzeitdaten einen numerischen Wert umfassen, der angibt, wie viel Feuchtigkeit im Blut eines aktuellen Benutzers der tragbaren Vorrichtung enthalten ist; und
die kumulativen Daten einen numerischen Wert umfassen, der angibt, wie viel Feuchtigkeit der Benutzer der tragbaren Vorrichtung für einen spezifizierten Zeitraum konsumiert hat.

8. Tragbare Vorrichtung nach einem der Ansprüche 4―7, wobei die gesammelten biometrischen Daten an eine Anwendung oder einen externen Dienstleister bereitgestellt werden.

9. Verfahren zur Messung des Hydratationsgrads einer Haut eines Benutzers unter Verwendung einer tragbaren Vorrichtung nach Anspruch 1, das von dem Prozessor der tragbaren Vorrichtung durchgeführt wird, wobei das Verfahren umfasst:
Steuern der Licht emittierenden Einheit zum Abstrahlen von Licht auf die Haut;
Steuern des Lichtteilers zum Teilen des Lichts;
Steuern (850) der Licht empfangenden Einheit zum Beziehen eines ersten Spektrums eines ersten Wellenlängenbands auf der Basis von mindestens einem Teil des Lichts, das gestreut und zurückgestrahlt wird, von dem Licht, das auf die Haut abgestrahlt wird;
Bestimmen eines ersten Hydratationsgrads, der mit der gesamten Haut assoziiert ist, auf der Basis des bezogenen ersten Spektrums;
Steuern (1110) der Licht empfangenden Einheit zum Beziehen eines zweiten Spektrums eines zweiten Wellenlängenbands auf der Basis von mindestens einem Teil des Lichts, das gestreut und zurückgestrahlt wird, von dem Licht, das auf die Haut abgestrahlt wird;
Bestimmen eines zweiten Hydratationsgrads, der mit einer Oberfläche der Haut assoziiert ist, auf der Basis des bezogenen zweiten Spektrums;
Bestimmen eines dritten Hydratationsgrads, der mit einer Dermis der Haut assoziiert ist, durch Subtrahieren des zweiten Hydratationsgrads von dem ersten Hydratationsgrad;
Sammeln von biometrischen Daten, die den dritten Hydratationsgrad beinhalten; und
Steuern der Anzeige zum Anzeigen, wodurch eine personalisierte Benutzeroberfläche (UI) auf der Anzeige bereitgestellt wird, auf der Basis der gesammelten biometrischen Daten.

10. Computerprogramm, umfassend Anweisungen, die dazu konfiguriert sind, bei Ausführen auf dem Prozessor der Vorrichtung nach Anspruch 1 zu bewirken, dass die Vorrichtung das Verfahren nach Anspruch 9 durchführt.

## Revendications

1. Dispositif portable (101, 201, 400, 700) comprenant :
un boîtier ;
une mémoire;
une unité émettrice de lumière (510) positionnée sur au moins une surface du boîtier et configurée pour irradier de la lumière ;
un diviseur de lumière (520) configuré pour diviser la lumière en au moins une bande de longueur d'onde ;
une unité de réception de lumière (530) positionnée sur la au moins une surface du boîtier et configurée pour recevoir au moins une partie de la lumière ;
un affichage (440) ; et
un processeur (410) connecté électriquement à au moins l'un parmi l'unité émettrice de lumière, le diviseur de lumière, l'unité de réception de lumière et l'affichage,
dans lequel le processeur (410) est configuré pour :
commander (810) l'unité émettrice de lumière pour irradier de la lumière vers la peau d'un utilisateur,
commander (830) le diviseur de lumière pour diviser la lumière,
commander (850) l'unité de réception de lumière pour obtenir un premier spectre d'une première bande de longueur d'onde sur la base d'au moins une partie de la lumière diffusée et renvoyée parmi la lumière irradiée vers la peau,
déterminer (870) un premier degré d'hydratation associé à l'ensemble de la peau à partir du premier spectre obtenu,
commander (1110) l'unité de réception de lumière pour obtenir un deuxième spectre d'une deuxième bande de longueur d'onde sur la base d'au moins une partie de la lumière diffusée et renvoyée parmi la lumière irradiée vers la peau,
déterminer (1130) un deuxième degré d'hydratation associé à une surface de la peau sur la base du deuxième spectre obtenu,
déterminer (1150) un troisième degré d'hydratation associé à un derme de la peau en soustrayant le deuxième degré d'hydratation du premier degré d'hydratation,
collecter (1410) des données biométriques incluant le troisième degré d'hydratation, et
commander (1430) l'affichage pour afficher une interface d'utilisateur personnalisée (UI) à l'utilisateur sur la base des données biométriques collectées.

2. Dispositif portable selon la revendication 1, dans lequel le processeur (410) est configuré pour :
identifier une quantité d'eau consommée par un utilisateur sur la base des données biométriques collectées, et
commander l'affichage pour afficher la quantité d'eau consommée par l'utilisateur dans l'interface d'utilisateur personnalisée.

3. Dispositif portable selon la revendication 1, dans lequel le diviseur de lumière est positionné à l'extrémité supérieure de l'unité émettrice de lumière ou de l'unité réceptrice de lumière.

4. Dispositif portable selon la revendication 1,
dans lequel le processeur est configuré pour commander la mémoire afin de collecter les données biométriques comprenant le troisième degré d'hydratation déterminé et pour commander l'affichage afin d'afficher l'interface d'utilisateur personnalisée (UI) à l'utilisateur sur la base des données biométriques collectées dans la mémoire.

5. Dispositif portable selon une quelconque des revendications précédentes, dans lequel le dispositif portable est configuré pour un ou plusieurs de la détermination d'au moins le premier degré d'hydratation :
lorsqu'une entrée manuelle d'un utilisateur est reçue lors de la saisie d'une application particulière de l'appareil portable ;
lorsqu'un événement particulier se produit ;
lorsqu'il est déterminé, d'après les informations biométriques de l'utilisateur, que l'utilisateur du dispositif portable se trouve dans une situation d'urgence ;
selon une période prédéfinie ; et
lorsque le dispositif portable est déterminé à s'approcher de la peau à une distance prédéterminée.

6. Dispositif portable selon une quelconque des revendications précédentes, dans lequel le dispositif portable est configuré pour collecter des données biométriques comprenant le troisième degré d'hydratation, dans un ou plusieurs de :
format de données en temps réel, ou
format de données cumulées.

7. Dispositif portable selon la revendication 6, dans lequel un ou plusieurs de:
les données en temps réel comprennent une valeur numérique indiquant la quantité d'humidité contenue dans le sang d'un utilisateur actuel du dispositif portable ; et
les données cumulées comprennent une valeur numérique indiquant la quantité d'humidité que l'utilisateur du dispositif portable a consommée pendant une période de temps spécifiée.

8. Dispositif portable selon une quelconque des revendications 4 à 7, dans lequel les données biométriques collectées sont fournies à une application ou à un fournisseur de services externe.

9. Procédé de mesure du degré d'hydratation d'une peau d'un utilisateur utilisant un dispositif portable selon la revendication 1, exécuté par le processeur de dispositif portable, le procédé comprenant :
la commande de l'unité émettrice de lumière pour irradier de la lumière vers la peau ;
la commande du diviseur de lumière pour diviser la lumière ;
la commande (850) de l'unité de réception de lumière pour obtenir un premier spectre d'une première bande de longueur d'onde sur la base d'au moins une partie de la lumière diffusée et renvoyée parmi la lumière irradiée vers la peau ;
la détermination d'un premier degré d'hydratation associé à l'ensemble de la peau d'après le premier spectre obtenu ;
la commande (1110) de l'unité de réception de lumière pour obtenir un deuxième spectre d'une deuxième bande de longueur d'onde sur la base d'au moins une partie de la lumière diffusée et renvoyée parmi la lumière irradiée vers la peau ;
la détermination d'un deuxième degré d'hydratation associé à une surface de la peau sur la base du deuxième spectre obtenu ;
la détermination d'un troisième degré d'hydratation associé à un derme de la peau en soustrayant le deuxième degré d'hydratation du premier degré d'hydratation ;
la collecte de données biométriques, y compris le troisième degré d'hydratation ; et
la commande de l'affichage à l'affichage fournissant une interface d'utilisateur personnalisée (UI) sur l'affichage sur la base des données biométriques collectées.

10. Programme informatique comprenant des instructions configurées pour, lorsqu'elles sont exécutées sur le processeur du dispositif selon la revendication 1, amener ledit dispositif à exécuter le procédé selon la revendication 9.
